# EUROPEAN PATENT APPLICATION

(11) **EP 4 555 976 A1**
(43) Date of publication of application: **21.05.2025**
(21) Application number: 23839821.8
(22) Date of filing: 19.06.2023
(51) Int. Cl.: A61F 2/14, A61F 9/00, A61F 9/007, A61L 27/54, A61L 27/48

(54) **IMPLANT DEVICE FOR EYE DISEASE TO WHICH DRUG DELIVERY MATRIX IS APPLIED**

(30) Priority: 15.07.2022 KR 20220087264
(71) Applicant: Microt Inc., Seoul 06351 (KR)
(72) Inventor: RYU, Junoh, Gwacheon-si Gyeonggi-do 13830 (KR); KIM, Jiyeon, Seoul 04775 (KR); CHOI, Seung Hyun, Seongnam-si Gyeonggi-do 13275 (KR); KO, Kyeonghyeon, Seoul 05544 (KR)
(74) Representative: Botti & Ferrari S.p.A.
(86) International application number: PCT/KR2023/008423
(87) International publication number: WO 2024/014727

(57) **Abstract**

An implant device for an eye disease to be inserted into an eyeball may include: a tube having one end to be inserted into an anterior chamber of the eyeball, the tube including a hollow portion through which aqueous humor is drained; and a matrix coupled to an outer surface of the tube and formed of a material capable of impregnating and releasing a drug. At least a part of the matrix may have a cross-section larger than a diameter of the tube. When the implant device for an eye disease is used, an antifibrotic agent, such as dexamethasone, mitomycin-C (MMC), 5-fluorouracil (5-FU), triamcinolone (TA), an anti-vascular endothelial growth factor (VEGF) inhibitor, or a transforming growth factor-beta-beta (TGF-β) inhibitor may be loaded on the matrix, and the antifibrotic agent may be released to surrounding tissue from the matrix after the implant device is inserted, thereby preventing an aqueous humor outlet of the tube from being blocked due to fibrosis.

## Description

### [Technical Field]

The present disclosure relates to an implant device for an eye disease, and more particularly, to an implant device for an eye disease in which a matrix configured to temporarily absorb a drug is coupled to a tube of the implant device, so that in a state where the implant device is inserted into an eyeball, the drug such as an antifibrotic agent is released from the matrix to surrounding tissue.

### [Background Art]

For a glaucoma patient whose intraocular pressure is not controlled even by using an intraocular pressure lowering agent, intraocular pressure is lowered by creating a bypass to drain aqueous humor from an anterior chamber of an eye to an external surface of the eye under the conjunctiva. Trabeculectomy among glaucoma filtration surgeries that create a bypass or a fistula for aqueous humor drainage may fail to control intraocular pressure when the amount of aqueous humor drainage decreases due to closure of the bypass after surgery. When initial surgery fails and glaucoma filtration surgery is performed again, the frequency of bypass closure after surgery increases and a success rate of surgery is low.

Also, even in the case of intractable glaucoma such as neovascular glaucoma or secondary glaucoma caused by uveitis according to types of glaucoma, closure of a bypass frequently occurs after trabeculectomy, resulting in poor results. For an eye with a history of failed glaucoma filtration surgery or intractable glaucoma, glaucoma implant surgery of locating a glaucoma implant device is performed to prevent closure of a bypass and increase a success rate of surgery. To date, glaucoma implant surgery has been used as an alternative to trabeculectomy, especially in several difficult-to-treat glaucoma, in that glaucoma implant surgery not only effectively lowers intraocular pressure but also shows a predictable postoperative clinical course according to an inner diameter of a given tube.

However, an existing glaucoma implant used for glaucoma implant surgery may cause various problems and complications such as difficulty in surgery due to a relatively large size, postoperative exposure, infection, eye movement disorder due to a large body, and diplopia. Accordingly, small-sized glaucoma implant instruments for minimally-invasive glaucoma surgery (MIGS) have recently been developed to relatively easily lower intraocular pressure by using a glaucoma implant and to reduce side effects after surgery due to a large size.

MIGS is a method of inserting a tube with a size of micrometers into an anterior chamber of an eye to drain aqueous humor from the anterior chamber. There is a possibility that the tube may become blocked due to fibrosis of tissue surrounding the eye at an aqueous humor outlet of the tube. When the aqueous humor outlet of the tube is blocked in this way, the aqueous humor may not be drained through the MIGS tube, causing a glaucoma patient's intraocular pressure to rise again. To solve this problem, reoperation is required to open a fibrotic area or re-insert an implant.

### [Disclosure]

### [Technical Problem]

According to one aspect of the present disclosure, an implant device for an eye disease may be provided, in which a matrix capable of impregnating and releasing a drug is coupled to a tube of the implant device, so that in a state where the implant device is inserted into an eyeball, the drug such as an antifibrotic agent is released from the matrix to surrounding tissue to prevent an aqueous humor outlet of the implant device from being blocked.

Also, according to one aspect of the present disclosure, an implant device for an eye disease may be provided, in which a matrix coupled to a tube of the implant device has a cross-section at least partially greater than a diameter of the tube inserted into an eyeball, so that when the tube is pushed into the eyeball after implant surgery, the matrix is caught on a sclera of the eyeball to prevent the tube from being completely inserted into the sclera.

### [Technical Solution]

According to an embodiment of the present disclosure, an implant device for an eye disease to be inserted into an eyeball includes a tube having one end to be inserted into an anterior chamber of the eyeball, the tube including a hollow portion through which aqueous humor is drained, and a matrix coupled to an outer surface of the tube and formed of a material capable of impregnating and releasing a drug. In this case, at least a part of the matrix has a cross-section larger than a diameter of the tube.

In an embodiment, the tube includes: a body extending in one direction; and a wing integrally formed with the body or detachably coupled to the body and extending in a lateral direction of the body. In this case, the matrix is at least partially coupled to the wing.

In an embodiment, the matrix includes a pair of sheet members. In this case, the tube is disposed between the pair of sheet members. In an embodiment, edges or vertices of the pair of sheet members may be at least partially adhered to each other.

In an embodiment, the matrix extends in a direction different from a longitudinal direction of the tube to prevent the tube from being completely inserted into a sclera of the eyeball.

In an embodiment, the matrix is coupled to the tube to protrude in a lateral direction of the tube at a position spaced apart from a distal end of the tube by a preset distance.

In an embodiment, the matrix includes any one material selected from the group consisting of poly(acrylic acid), polyacrylamide, poly(sulfopropyl acrylate), poly(2-hydroxyethyl methacrylate), poly(vinyl alcohol), silicone, polyurethane, collagen, gelatin, hyaluronic acid, poly(aspartic acid), alginate, hydroxypropyl cellulose, hydroxypropyl methylcellulose acetate succinate, and chitosan, or a combination thereof.

In an embodiment, the matrix includes a first material that is not biodegradable in the eyeball and a second material capable of temporarily absorbing a drug.

In an embodiment, the first material includes any one material selected from the group consisting of silicone, polyethylene vinyl acetate, polyvinyl acetate, polycarbonate, polyvinyl chloride, polyurethane, poly(methyl methacrylate), poly(butyl methacrylate), polyethylene, polypropylene, polyethylene terephthalate (PET), glycol-modified PTE, PTFE, polyhydroxyalkanoates, parylene, polyether ether ketone, polyimide, epoxy-based resin such as SU-/, poly(vinylidene fluoride), polyether block amides, 3-[tris(trimethylsiloxy)silyl]propyl methacrylate, poly(styrene-b-isobutylene-b-styrene) copolymer, cellulose acetate, poly((N-vinylpyrrolidone)-block-poly(vinyl acetate)), and ethyl cellulose, or a combination thereof.

Also, in an embodiment, the second material includes any one material selected from the group consisting of poly(ethylene glycol) (PEG), PEG derivatives, poly(ethylene oxide), poly(propylene oxide), polyvinyl alcohol, polyvinyl pyrrolidone, polyethersulfone, polyamide, polyacrylamide, polyglycolic acid, polyacrylic acid, glucuronic acid, hexuronic acid, hyaluronic acid, polyaspartic acid, alginate, polyorthoester, 2-hydroxyethyl methacrylate, polycaprolactone (PCL), polylactic acid (PLA), poly(lactide-co-glycolide) (PLGA), polydioxanone (PDO), polyhydroxybutyrate (PHB), polyhydroxyalkanoates (PHA), collagen, gelatin, hydroxypropyl cellulose, and chitosan, or a combination thereof.

In an embodiment, the matrix is configured to impregnate a drug including at least one material selected from the group consisting of dexamethasone, mitomycin-C (MMC), 5-fluorouracil (5-FU), triamcinolone (TA), an anti-vascular endothelial growth factor (VEGF) inhibitor, and a transforming growth factor-beta (TGF-β) inhibitor, or a combination thereof.

### [Advantageous Effects]

According to an implant device for an eye disease according to an embodiment of the present disclosure, because a matrix formed of a material capable of temporarily absorbing a drug and capable of impregnating and releasing the drug is coupled to a tube of the implant device, in a state where the implant device is inserted into an eyeball, the drug may be released to surrounding tissue through the matrix.

For example, when an antifibrotic agent such as dexamethasone, mitomycin-C (MMC), 5-fluorouracil (5-FU), triamcinolone (TA), an anti-vascular endothelial growth factor (VEGF) inhibitor, or a transforming growth factor-beta (TGF-β) inhibitor is loaded on the matrix, because the antifibrotic agent is released from the matrix coupled to the tube to tissue around an aqueous humor outlet of the tube, the aqueous humor outlet may be prevented from being blocked due to fibrosis of the tissue around the tube.

Also, in the implant device for an eye disease according to an embodiment of the present disclosure, because a cross-section of the matrix is at least partially greater than a diameter of the tube, even when the tube is pushed into the eyeball due to any cause after surgery using the implant device, the matrix with a relatively large cross-section is caught on a sclera of the eyeball, thereby preventing the tube from being completely inserted into the sclera of the eyeball.

### [Description of Drawings]

FIGS. 1 and 2 are conceptual views illustrating a state where an implant device for an eye disease is inserted into an eyeball according to an embodiment.
FIG. 3A is a perspective view illustrating an implant device for an eye disease according to an embodiment.
FIG. 3B is a perspective view illustrating an implant device for an eye disease according to another embodiment.
FIGS. 4A and 4B are perspective views illustrating an arrangement of a tube and a matrix in an implant device for an eye disease according to embodiments.
FIG. 5 is a conceptual view illustrating a process of inserting an implant device for an eye disease into an eyeball by using an injector according to an embodiment.
FIGS. 6A to 6E are perspective views illustrating an implant device for an eye disease according to still other embodiments.
FIG. 7A is a perspective view illustrating a tube of an implant device for an eye disease according to still another embodiment.
FIG. 7B is a cross-sectional view illustrating the tube of FIG. 7A.
FIG. 8 is a cross-sectional view illustrating a shape of a wing in an implant device for an eye disease according to embodiments.
FIG. 9 is a plan view illustrating a shape of a wing in an implant device for an eye disease according to embodiments.
FIG. 10 is a plan view illustrating a shape of a matrix in an implant device for an eye disease according to embodiments.

### [Mode for Invention]

The terms used herein will be briefly described, and the present disclosure will be described in detail.

The terms used herein are general terms currently widely used in the art in consideration of functions in the present disclosure, but the terms may vary according to the intention of one of ordinary skill in the art, precedents, or new technology in the art. Also, some of the terms used herein may be arbitrarily chosen by the present applicant, and in this case, these terms are defined in detail below. Accordingly, the specific terms used herein should be defined based on the unique meanings thereof and the whole context of the present disclosure.

It will be understood that when a certain part "includes" a certain component, the part does not exclude another component but may further include another component, unless the context clearly dictates otherwise. Also, throughout the specification, when an element is referred to as being "connected" to another element, it will be understood to include that the element is "directly connected" to the other element or is "connected" to the other element with another element therebetween.

The present disclosure will now be described more fully with reference to the accompanying drawings for one of ordinary skill in the art to be able to perform the present disclosure without any difficulty. However, the present disclosure may be embodied in many different forms and is not limited to the embodiments set forth herein. For clarity, portions irrelevant to the descriptions of the present disclosure are omitted in the drawings, and like components are denoted by like reference numerals throughout the specification.

Hereinafter, the present disclosure will be described in detail with reference to the accompanying drawings.

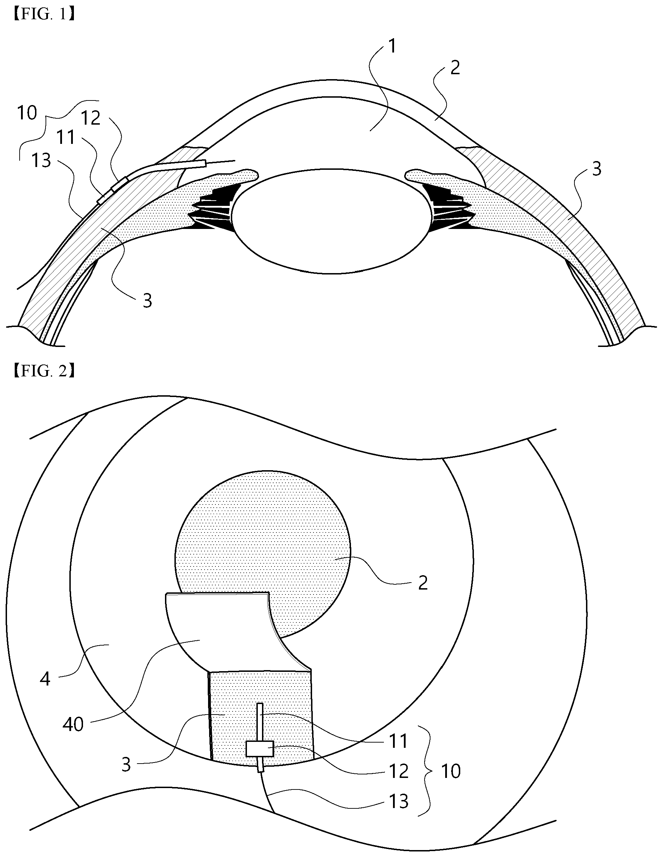

FIGS. 1 and 2 are conceptual views illustrating a state where an implant device for an eye disease is inserted into an eyeball according to an embodiment.

Referring to FIGS. 1 and 2, an implant device 10 for an eye disease according to embodiments is intended to control intraocular pressure by controlling the amount of aqueous humor drainage in an anterior chamber 1 located in front of a lens and below a cornea 2 in an eyeball, thereby preventing damage to an optic nerve due to increased intraocular pressure caused by eye diseases. The implant device 10 for an eye disease according to embodiments of the present disclosure may be used to treat or alleviate symptoms of various eye diseases that cause or are caused by increased intraocular pressure.

Eye diseases in the specification may include glaucoma caused by an increase in intraocular pressure. Examples of glaucoma may include, but are not limited to, congenital glaucoma, traumatic glaucoma, glaucoma suspect, ocular hypertension, primary open-angle glaucoma, normal-tension glaucoma, capsular glaucoma with pseudoexfoliation of lens, chronic simple glaucoma, low-tension glaucoma, pigmentary glaucoma, primary angle-closure glaucoma, acute angle-closure glaucoma, chronic angle-closure glaucoma, intermittent angle-closure glaucoma, glaucoma secondary to eye trauma, glaucoma secondary to eye inflammation, glaucoma secondary to drugs, neovascular glaucoma, and secondary glaucoma due to uveitis.

The implant device 10 for an eye disease according to embodiments may include a tube 11 applicable to minimally-invasive glaucoma surgery (MIGS), and one end of the tube 11 may be inserted into an anterior chamber 1 of an eyeball and the other end of the tube 11 may be located on conjunctival tissue or Tenon's tissue 4. The tube 11 may include a hollow portion through which aqueous humor of the eyeball may flow, and may allow the aqueous humor to be drained from the anterior chamber 1 of the eyeball to the outside of a sclera 3 of the eyeball through the tube 11.

Referring to FIG. 2, the implant 10 for an eye disease may be inserted after an operator exfoliates the conjunctival tissue or Tenon's tissue 4 of the eyeball, and after insertion, the implant device 10 may be placed in the eyeball by covering the conjunctival tissue or Tenon's tissue 4 again. That is, after a flap 40 is created by cutting a part of the conjunctival tissue or Tenon's tissue 4, the created flap 40 may be lifted and the distal end of the tube 11 may be inserted to pass through the exposed sclera 3 of the eyeball. In this case, the tube 11 is not completely inserted into the sclera 3, and a proximal end of the tube 11 may be located on or inserted into the conjunctival tissue or Tenon's tissue 4. After one end of the tube 11 is inserted into the anterior chamber 1 through the sclera 3, a process of inserting the implant device 10 for an eye disease is completed by lowering the lifted flap 40.

Also, the implant device 10 for an eye disease according to embodiments further includes a matrix 12 coupled to a portion of the tube 11 and formed of a material capable of impregnating and releasing a drug. In the specification, the matrix 12 refers to a release control material that delivers a drug to be administered to the eyeball by using the implant device 10 for an eye disease to tissue surrounding the eyeball into which the implant device 10 for an eye disease is inserted. That is, the matrix 12 temporarily absorbs the drug and then releases the drug to the tissue surrounding the eyeball in a state where the implant device 10 for an eye disease is inserted into the eyeball.

In the specification, the matrix 12 may have hydrophobicity or hydrophilicity according to a type of a drug to be delivered through the matrix 12, and may be formed by mixing two or more materials having different hydrophilicity-related characteristics. Also, it is preferable that the matrix 12 is formed of a material that is biocompatible and is not immediately decomposed in the eyeball, and is formed of a material that may release the drug continuously (e.g., for several days or more) in a state where the implant device 10 for an eye disease is inserted into the eyeball.

In the specification, when a certain material is described as not immediately decomposed or biodegraded, it means that the material is not affected or hardly affected by a biological environment surrounding the specific material and undergoes no or very little structural change over a treatment period using the implant device according to embodiments. That is, even when a material is decomposed in a human body over a very long period of time exceeding the treatment period of the eye disease, the material may correspond to a material that is not immediately decomposed or biodegraded in the specification.

The matrix 12 has a cross-section that is at least partially larger than a diameter of the tube 11. As a result, the matrix 12 may extend and/or protrude in a lateral direction different from a longitudinal direction of the tube 11. The matrix 12 may be formed as a sheet covering a part of an outer surface of the tube 11, or may be formed as a plurality of sheets located with the tube 11 therebetween. Also, when the matrix 12 includes a plurality of sheets, the sheets may be at least partially adhered to each other.

In an embodiment, the implant device 10 for an eye disease further includes a ripcord 13 inserted into the hollow portion of the tube 11. The ripcord 13 is for controlling pressure formed in the anterior chamber through the hollow portion of the tube 11. When the ripcord 13 is inserted into the hollow portion of the tube 11, the flow of the aqueous humor becomes unsmooth, causing the aqueous humor to be accumulated in the anterior chamber of the eye, and intraocular pressure is relatively increased compared to when the ripcord 13 is not present. In the specification, pressure formed in the anterior chamber refers to pressure in the anterior chamber of the eyeball formed at this time. The implant device10 for an eye disease may be inserted into the eyeball with the ripcord 13 inserted into the tube 11, or the implant device 10 for an eye disease, excluding the ripcord 13, may be inserted into the eye and then the ripcord 13 may be inserted into the tube 11.

In an embodiment, in the implant device 10 for an eye disease, an implant body (not shown) may be further coupled to the proximal end of the tube 11 located in the conjunctival tissue or Tenon's tissue 4 of the eyeball. The implant body may include one or more membranes arranged to surround an end of the tube 11, and each membrane may be formed of, for example, a urethane-based material such as polytetrafluoroethylene (PTFE) or polycarbonate-polyurethane (PC-PU), a silicone-based material such as polydimethylsiloxane (PDMS), a siloxane/polyurethane compound (siloxane-based polyurethane), polyethylene, polypropylene, or polymethyl methacrylate (PMMA).

The implant body is coupled to the rear of the tube 11 and temporarily accommodates the aqueous humor to effectively control intraocular pressure. For example, the implant body may be coupled and located at the rear of the tube 11 through the exfoliated conjunctival tissue or Tenon's tissue 4 in consideration of a clinical situation such as a rate of fibrosis or a change in a patient's condition after the tube 11 of the implant device 10 for an eye disease is inserted into the anterior chamber 1 of the eyeball.

Alternatively, according to an embodiment, the tube 11 and the implant body may be coupled to each other or integrally formed with each other and may be placed together into the eyeball through the exfoliated conjunctival tissue or Tennon's tissue 4 of the eyeball. That is, the implant body may be pre-coupled to the tube 11 before the implant device is placed in the eyeball, or the tube 11 may be first placed in the eyeball and then may be coupled to the implant body, according to clinical needs or circumstances.

When the implant device 10 for an eye disease is inserted into the eyeball, the aqueous humor generated in the anterior chamber may flow through the tube 11 of the implant device for an eye disease, thereby draining the aqueous humor from the anterior chamber and lowering intraocular pressure. When the implant body is coupled to the tube 11, the aqueous humor drained from the anterior chamber may be temporarily accommodated in the implant body. When the amount of the aqueous humor accommodated in the implant body exceeds a certain amount, the accommodated aqueous humor may be drained to the conjunctival tissue or Tennon's tissue 4 through the rear of the implant body, thereby effectively controlling intraocular pressure.

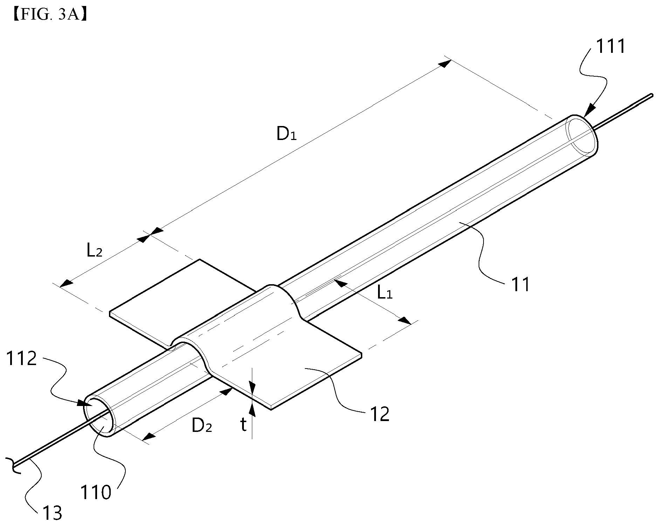

FIG. 3A is a perspective view illustrating an implant device for an eye disease according to an embodiment.

Referring to FIG. 3A, the implant device for an eye disease according to the present embodiment includes the tube 11 applicable to MIGS and the matrix 12 coupled to the tube 11. The matrix 12 may be coupled to an outer surface of a portion of the tube 11, and may be permanently coupled to the tube 11 or detachably fixed to the tube 11. In an embodiment, the matrix 12 may be coupled to a surface of the tube 11 by using an adhesive material.

The tube 11 is inserted into an eyeball so that a distal end 111 is located in an anterior chamber of the eyeball and a proximal end 112 is located on conjunctival tissue or Tenon's tissue of the eyeball, and the tube 11 allows aqueous humor generated in the anterior chamber of the eyeball to be drained through a hollow portion 110 in the tube 11 to the conjunctival tissue or the Tennon's tissue. In the specification, the distal end 111 and the proximal end 112 are defined according to a direction from an operator inserting the implant device, and among both ends of the tube 11, the proximal end 112 refers to an end facing the operator and the distal end 111 refers to an end facing a patient's eye into which the implant device is to be inserted.

In an embodiment, the tube 11 may be formed of a biocompatible material and may be formed of a changeable material. For example, the tube 11 may be formed of silicone or other silicone-based material, a urethane-based material such as PTFE, polycarbonate, or polyurethane (PU), a composite material of a silicone-based material and a polyurethane-based material such as silicone-PU, or a biocompatible metal or alloy.

In an embodiment, the tube 11 may be formed of, but is not limited to, silicone, PTFE, polycarbonate, polyurethane, polyethylene, polypropylene, polyimide, PMMA, poly(styrene-b-isobutylene-b-styrene) copolymer, polyethersulfone, gelatin, stainless steel, titanium, nitinol, or a combination thereof.

In an embodiment, the tube 11 may be formed in a curved shape with a certain curvature in order to prevent damage to the endothelium of a cornea in the eyeball. A front end of the tube may poke and damage the cornea in the anterior chamber of the eyeball in a process of inserting the tube into the anterior chamber of the eyeball according to a size of the eyeball that is different for each patient, a skill level in tube injection, etc. Damage to the cornea may cause complications such as corneal decompensation which require even future corneal transplantation. According to the present embodiment, in order for the tube to naturally move in a curved shape while being inserted into the anterior chamber of the eyeball, the tube 11 may be manufactured in a curved shape with a certain curvature corresponding to a curvature of a surface of the eyeball.

The matrix 12 is coupled to an outer surface of the tube 11, and at least a portion of the matrix 12 has a cross-section larger than a diameter of the tube 11. As a result, a part of the matrix 12 protrudes in a lateral direction of the tube 11, that is, in a direction different from a longitudinal direction of the tube 11. A length L₁ of the matrix 12 extending in the lateral direction of the tube 11 and a length L₂ of the matrix 12 along the longitudinal direction of the tube 11 may be appropriately determined so that when the tube 11 is pushed toward a sclera of the eyeball, the matrix 12 is caught on the sclera to prevent movement of the tube 11.

In the embodiment of FIG. 3A, the matrix 12 is disposed on the tube 11 to partially cover a top surface of the tube 11. For example, when the tube 11 is inserted into the sclera of an eyeball, the matrix 12 coupled to the tube 11 may be placed on the sclera while covering a part of the tube 11. However, this is only an example, and in another embodiment, the matrix 12 may be disposed on a bottom surface of the tube 11.

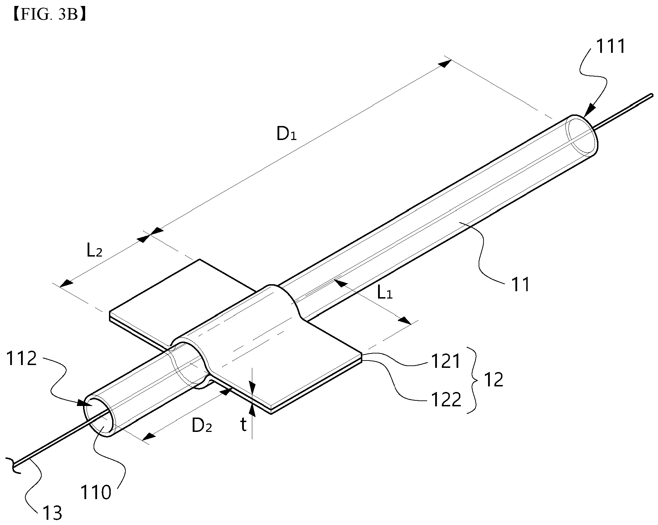

Also, in an embodiment, the matrix 12 may include a pair of sheet members 121, 122 as in the embodiment of FIG. 3B, and the pair of sheet members 121, 122 may be located over and under the tube 11 with the tube 11 therebetween. The matrix 12 may be coupled to the tube 11 by adhering corners and vertices of the sheet members 121, 122 to each other in a state where the tube 11 is inserted between the pair of sheet members 121, 122. For example, the pair of sheet members 121, 122 may be adhered to each other by using an adhesive material such as a silicone adhesive. Also, in an embodiment, any one or both the sheet members 121, 122 may be adhered to the surface of the tube 11 by using an adhesive material.

The matrix 12 is formed of a material capable of impregnating a drug such as an antifibrotic agent, and is a release control material capable of releasing the absorbed drug to surrounding tissue in the eyeball in a state where the implant device is inserted into the eyeball. For example, in a state where the matrix 12 containing no drug is immersed in a drug in a liquid state so that the drug permeates into the matrix 12, the implant device including the tube 11 to which the matrix 12 is coupled may be inserted into the patient's eyeball. However, this is only an example, and in another embodiment, the matrix 12 may be manufactured in a state where a drug is loaded by mixing the drug in a dispersion solution for preparing a polymer.

In an embodiment, the tube 11 and the matrix 12 constituting the implant device 10 for an eye disease may be coupled to each other in a process of inserting the implant device. For example, the operator may insert the tube 11 of the implant device into the patient's sclera, and then may couple the tube 11 to the matrix 12 by placing the matrix 12 on the surface of the tube 11 exposed to the outside of the sclera. In this case, the matrix 12 may be manufactured with a drug preloaded, or the operator may immerse the matrix 12 containing no drug in a drug in an operating room so that the drug permeates into the matrix 12 and may place the matrix 12 on the tube 11. Also, the operator may couple the tube 11 to the matrix 12 by passing a suture thread through the matrix 12 and fixing to ocular tissue.

Examples of a drug to be loaded on the matrix 12 may include antifibrotic agents such as, but not limited to, dexamethasone, mitomycin-C (MMC), 5-fluorouracil (5-FU), triamcinolone (TA), an anti-vascular endothelial growth factor (anti-VEGF) inhibitor, and a transforming growth factor-beta (TGF-β) inhibitor. For example, according to a purpose of applying a drug to the matrix 12, in addition to an antifibrotic agent, an intraocular pressure lowering agent such as prostaglandin, ceftazidime or another cefa-based antibiotic, a fluoroquinolone-based antibiotic such as levofloxacin or moxifloxacin, another type of antibiotic, or another type of drug may be loaded on the matrix 12.

The matrix 12 may be formed of a material that is not immediately decomposed in a living body, allowing sufficient time for the drug to be released, and does not expand excessively enough to cause foreign body sensation in the eye due to the drug. In an embodiment, a polymer having a membrane structure capable of forming a storage space for capturing the drug may be used as the matrix 12.

For example, the matrix 12 may be formed of, but is not limited to, poly(acrylic acid), polyacrylamide, poly(sulfopropyl acrylate), poly(2-hydroxyethyl methacrylate), poly(vinyl alcohol), silicone, polyurethane, collagen, gelatin, hyaluronic acid, poly(aspartic acid), alginate, hydroxypropyl cellulose, hydroxypropyl methylcellulose acetate succinate, chitosan, or a combination thereof.

In an embodiment, the matrix 12 may be a material having a double-layer structure formed of two or more different materials. In this case, a first material of the matrix 12 may be a material having biocompatibility and mechanical properties for forming the skeleton of the matrix 12 without being immediately biodegraded in the eyeball. Also, a second material, which is another material constituting the matrix 12 together with the first material, may be a material that may temporarily absorb a drug when combined with the first material. When the matrix 12 includes a composite of the first material and the second material as in the present embodiment, a drug suitable for the purpose may be easily impregnated in the matrix 12 while maintaining the mechanical strength of the matrix 12. For example, the matrix 12 may be formed as a sheet including a composite of PU and PEG.

In an embodiment, the first material may be, but is not limited to, silicone, polyethylene vinyl acetate, polyvinyl acetate, polycarbonate, polyvinyl chloride, polyurethane, PMMA, poly(butyl methacrylate), polyamide, polyethylene, polypropylene, polyethylene terephthalate (PET), glycol-modified PTE, PTFE, polyhydroxyalkanoates, parylene, polyether ether ketone, polyimide, epoxy-based resin such as SU-8, poly(vinylidene fluoride), polyether block amides, 3-[tris(trimethylsiloxy)silyl]propyl methacrylate, poly(styrene-b-isobutylene-b-styrene) copolymer, cellulose acetate, poly((N-vinylpyrrolidone)-block-poly(vinyl acetate)), ethyl cellulose, or a combination thereof.

Also, in an embodiment, the second material may be, but is not limited to, poly(ethylene glycol) (PEG), PEG derivatives, poly(ethylene oxide), poly(propylene oxide), polyvinyl alcohol, polyvinyl pyrrolidone, polyethersulfone, polyamide, polyacrylamide, polyglycolic acid, polyacrylic acid, glucuronic acid, hexuronic acid, hyaluronic acid, polyaspartic acid, alginate, polyorthoester, 2-hydroxyethyl methacrylate, polycaprolactone (PCL), polylactic acid (PLA), poly(lactide-co-glycolide) (PLGA), polydioxanone (PDO), polyhydroxybutyrate (PHB), polyhydroxyalkanoates (PHA), collagen, gelatin, hydroxypropyl cellulose, chitosan, or a combination thereof.

In an embodiment, the matrix 12 may be coupled to the tube 11 to be spaced apart from the distal end 111 and the proximal end 112 of the tube 11 by certain distances D₁, D₂, respectively. When a drug released from the matrix 12 is an antifibrotic agent, the matrix 12 may be disposed adjacent to the proximal end 112 of the tube through which the aqueous humor is drained in order to prevent an aqueous humor outlet from being blocked due to fibrosis of tissue. That is, in the present embodiment, the distance D₁ between the distal end 111 of the tube 11 and the matrix 12 may be greater than the distance D₂ between the proximal end 112 of the tube 11 and the matrix 12.

Also, a thickness t of the matrix 12 may be small enough to prevent the patient from feeling a foreign matter even when the matrix 12 is inserted into the conjunctival tissue or the Tenon's tissue. When the matrix 12 includes a plurality of sheet members 121, 122 as in the embodiment of FIG. 3B, the total thickness t of the plurality of sheet members 121, 122 may be determined to be small enough to prevent the patient from feeling a foreign matter. Also, in an embodiment, the thickness t of the matrix 12 may be less than a diameter of the tube 11.

In an embodiment, the implant device for an eye disease further includes the ripcord 13 at least partially inserted into the hollow portion 110 of the tube 11. The ripcord 13 may be a non-absorbable surgical suture thread, and may be formed of a nylon or prolene material, but the present disclosure is not limited thereto.

The ripcord 13 inserted into the hollow portion 110 of the tube 11 controls pressure formed in the anterior chamber. When the ripcord 13 is thick, a space between an inner wall of the tube 11 and the ripcord 13 becomes narrow and the aqueous humor is relatively slowly drained, and thus, pressure formed in the anterior chamber increases. When the ripcord 13 is thin, a space between the inner wall of the tube 11 and the ripcord 13 becomes wide and the aqueous humor is relatively rapidly drained, and thus, pressure formed in the anterior chamber decreases. Accordingly, pressure formed in the anterior chamber may be optimized to be within a certain range, for example, postoperative pressure of about 6 mmHg to about 21 mmHg, through an appropriate configuration of the ripcord 13. However, a preferred numerical range of pressure formed in the anterior chamber is not limited thereto.

In an embodiment, the ripcord 13 may be manipulated by a clinician to control pressure formed in the anterior chamber. For example, when the ripcord 13 is inserted into the hollow portion 110 of the tube 11 and exposed from a rear end of the tube 11, the clinician may control the amount of aqueous humor drainage by adjusting the ripcord 13 exposed from the rear end of the tube 11. That is, the clinician may appropriately control intraocular pressure according to the patient's condition by using the ripcord 13. In the present embodiment, in order to prevent a user from feeling a foreign matter due to the ripcord 13 exposed to the outside of the tube 11, the ripcord 13 may be formed to gradually decrease in diameter from a point where the ripcord 13 is exposed from the tube 11 or an implant body (not shown) coupled to the rear end of the tube 11.

In the embodiment described with reference to FIGS. 3A and 3B, the matrix 12 is spaced apart from the proximal end 112 of the tube 11 by the certain distance D₂. However, this is only an example, and in another embodiment, the matrix 12 may be disposed in contact with the proximal end 112 of the tube 11 (i.e., the distance D₂ is 0), or may further extend beyond the proximal end 112 of the tube 11 away from the eyeball (i.e., the distance D₂ is negative).

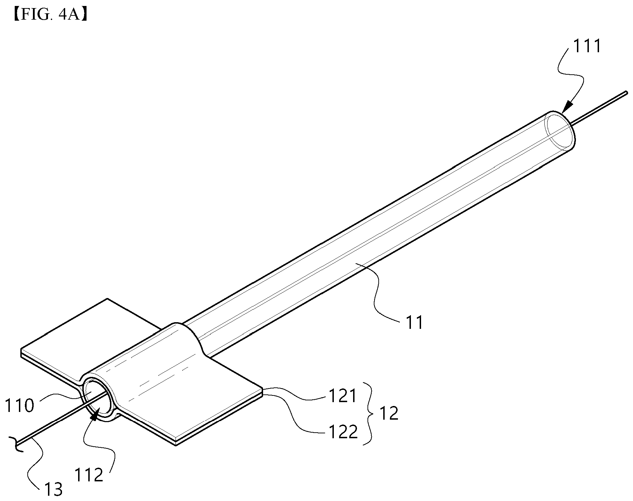

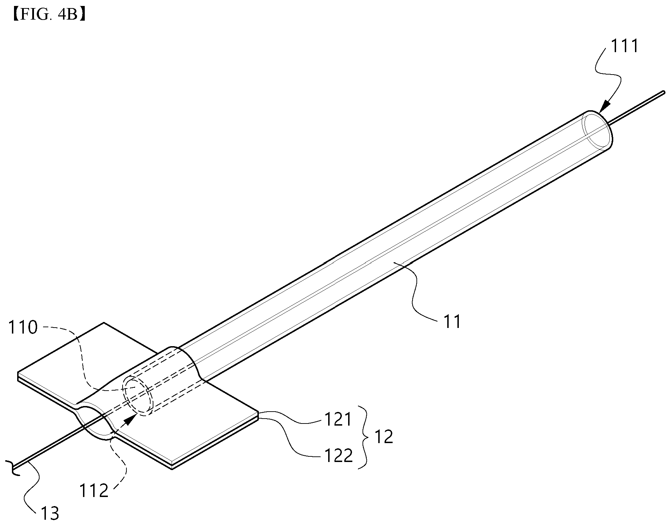

FIGS. 4A and 4B are perspective views illustrating an arrangement of a tube and a matrix in an implant device for an eye disease according to embodiments. Although an arrangement of the matrix 12 including one pair of sheet members 121, 122 is illustrated in FIGS. 4A and 4B, the same arrangement may be applied to the matrix 12 including a single sheet as shown in FIG. 3A.

FIG. 4A illustrates an example where the matrix 12 is coupled to the tube 11 so that one end of the matrix12 is completely aligned with the proximal end 112 of the tube. In this case, because a drug such as antifibrotic agent is drained by the matrix 12 at a position that is in direct contact with the proximal end 112 through which aqueous humor is drained, an aqueous humor outlet may be prevented from being blocked due to fibrosis of ocular tissue.

Also, FIG. 4B illustrates an example where one end of the matrix 12 extends beyond the proximal end 112 of the tube 11 in a direction (i.e., a direction away from an eyeball or a direction toward an operator). In the present embodiment, because the matrix 12 completely surrounds the proximal end 112 of the tube 11 and the aqueous humor drained from the tube 11 is drained after passing through an area covered by the matrix 12, when an antifibrotic agent is impregnated in the matrix 12, the aqueous humor outlet may be fundamentally prevented from being blocked due to fibrosis.

In the embodiment of FIG. 4B, the matrix 12 may be formed of a flexible material, and thus, a shape of the aqueous humor outlet may not be fixedly supported in a portion where the matrix 12 extends beyond the proximal end 112 of the tube 11. That is, when the matrix 12 includes a single sheet, the matrix 12 may cover the proximal end 112 of the tube 11 without a fixed aqueous humor outlet, or when the matrix 12 includes a plurality of sheets, the sheets facing each other may be attached to each other without a fixed aqueous humor outlet.

However, when the aqueous humor is drained from the proximal end 112 through the tube 11 and pressure of the drained aqueous humor reaches a certain level, the aqueous humor outlet passing through the matrix 12 may be naturally formed due to the pressure of the aqueous humor, thereby draining the aqueous humor. That is, when the amount of aqueous humor drained through the tube 11 is a certain amount or more, the aqueous humor may push the matrix 12 and may be drained to the outside of the matrix 12. For example, when the matrix 12 includes a sheet covering the proximal end 112 of the tube 11, if a certain amount of aqueous humor is collected, the aqueous humor may lift the matrix 12 to form a passage through which a fluid flows.

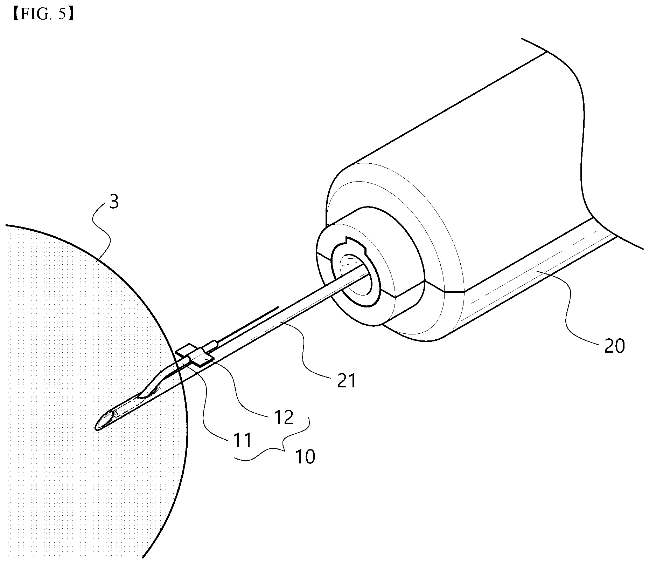

FIG. 5 is a conceptual view illustrating a process of inserting an implant device for an eye disease into an eyeball by using an injector according to an embodiment.

Referring to FIG. 5, the implant device for an eye disease according to embodiments may be injected into an eyeball through an injector 20 including a needle 21, so that one end of the tube 11 is inserted into an anterior chamber through the sclera 3 of the eyeball as shown in FIGS. 1 and 2. Here, the injector 20 refers to an arbitrary instrument used to insert the implant device into the eyeball by mechanically moving the needle 21 or the injector 20 forward in a state where the implant device is accommodated in the needle 21 or is fixed to the needle 21 by using another method.

For example, in a state where the implant device is loaded in the needle 21, an operator may move the entire injector 20 forward so that one end of the tube 11 of the implant device is inserted into the anterior chamber through the sclera 3, and then with the implant device in the inserted position, the operator may move only the needle 21 backward through an arbitrary mechanism provided in the injector 20 or move the entire injector 20 backward so that the implant device is inserted into the eyeball. Because the injector 20 for inserting the implant device may be any of various types well known or to be developed in the future, a detailed description of the injector 20 will be omitted in the specification to clarify the gist of the present disclosure.

Meanwhile, as the implant device for an eye disease according to embodiments includes the matrix 12 having a cross-section larger than a diameter of the tube 11, the matrix 12 may be inserted into the needle 21 of the injector 20 together with the tube 11, or the implant device may be inserted into a patient's eye in a state where the matrix 12 is located outside the needle 21.

For example, in an embodiment, the matrix 12 has a cross-section larger than a diameter of the tube 11 but may be rolled or folded because the matrix 12 is formed of a flexible material. Accordingly, the matrix 12 may be inserted into the needle 21 together with the tube 11 while being rolled or folded to be in close contact with a surface of the tube 11, and when the tube 11 reaches an insertion position, the matrix 12 may be unfolded while exiting the needle 21 together with the tube 11. Alternatively, in a state where the matrix 12 is not inserted into the needle 21 and only a front end of the tube 11 not coupled to the matrix 12 is inserted into the needle 21 or fixed to the needle 21, the implant device may be placed in the eyeball in such a way that the needle 21 pulls the matrix 12 together through the front end of the tube 11.

However, a method of arranging the tube 11 and the matrix 12 is not limited to the examples described in the specification. Any other clinical method may be used. For example, the matrix 12 may be coupled to the tube 11 through an additional procedure in a state where only the tube 11 is inserted into the eyeball by using the injector 20.

As described above, the matrix 12 in the implant device according to embodiments impregnates a drug such as an antifibrotic agent and slowly releases the drug to ocular tissue. The tube 11 is for MICS and usually has a micrometer-level diameter, and thus, there is a possibility that the tube 11 may be pushed into the sclera 3 due to impact or other causes even after the tube 11 is inserted into the sclera. However, according to embodiments of the present disclosure, because the matrix 12 having a cross-section larger than a diameter of the tube 11 is coupled to the tube 11, even when the tube 11 moves toward the sclera 3, the matrix 12 may be caught on the sclera 3 and the tube 11 may be prevented from being completely inserted into the sclera 3.

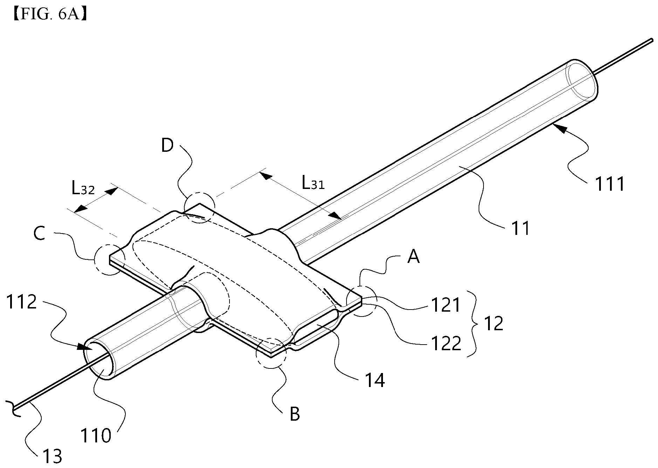

FIG. 6A is a perspective view illustrating an implant device for an eye disease according to still another embodiment.

Referring to FIG. 6A, the implant device for an eye disease according to the present embodiment includes a wing 14 extending in a lateral direction of a body of the tube 11. In the embodiment of FIG. 6A, the tube 11 of the implant device 11 is inserted into another tube having a larger cross-sectional diameter, so that a part of the outer tube surrounding the tube 11 forms the wing 14.

However, this is only an example, and a method of forming a wing extending in the lateral direction of the tube 11 is not limited thereto. For example, the implant device may be configured so that a separate member functioning as a wing may be coupled to a surface of the tube 11 or the tube 11 itself includes a specific portion with an enlarged outer diameter and functioning as a wing. Furthermore, in an embodiment, a wing formed on a side surface of the tube 11 may be integrally formed with the tube 11 or may be detachably formed on the tube 11.

In the present embodiment, the matrix 12 coupled to the tube 11 may be at least partially coupled to the wing 14 on the side surface of the tube 11. In an embodiment, the matrix 12 may include one pair of sheet members 121, 122, and the matrix 12 may be coupled to the wing 14 by adhering the sheet members 121, 122 to each other in a state where the wing 14 of the tube 11 is disposed between the sheet members 121, 122. In the specification, the sheet members may be adhered by using an adhesion method using a silicone adhesive or the like.

For example, in the embodiment of FIG. 6A, the upper and lower sheet members 121, 122 each have a quadrangular shape, and are adhered to each other at vertices A, B, C, D of the quadrangular shape, and the tube 11 or the wing 14 is located at an edge portion between adhered portions of the sheet members 121, 122. That is, the tube 11 extends through edges AD and BC of the sheet members 121, 122 having the quadrangular shape, and the wing 14 orthogonal to a longitudinal direction of the tube 11 extends through edges AB and CD of the sheet members 121, 122. A length L₃₁ of the wing 14 extending in the lateral direction of the tube 11 and a length L₃₂ of the wing 14 along the longitudinal direction of the tube 11 may be appropriately determined so as not to hinder adhesion between the upper and lower sheet members 121, 122 due to the presence of the wing 14.

In the present embodiment, the wing 14 of the tube 11 provides coupling stability with the matrix 12. In the coupled state shown in FIG. 6A, because the upper and lower sheet members 121, 122 are adhered to each other at the vertices A, B, C, D and the wing 14 of the tube 11 is located therebetween, even when an external force is applied to the tube 11 back and force in the longitudinal direction, the wing 14 is fixed between the upper and lower sheet members 121, 122 and thus, the matrix 12 is not separated from the tube 11. In an embodiment, the matrix 12 and the wing 14 may be adhered to each other by using an adhesive such as a silicone adhesive for coupling stability.

Also, according to the present embodiment, because the matrix 12 having a larger diameter than the tube 11 protrudes on the side surface of the tube and the tube 11 itself includes the wing 14 protruding in the lateral direction to form a stepped portion, after the implant device is inserted, the tube 11 may be physically prevented from being excessively inserted into the eyeball and, after the tube 11 is inserted, the tube 11 may be prevented or minimized from moving back and forth due to the stepped portion.

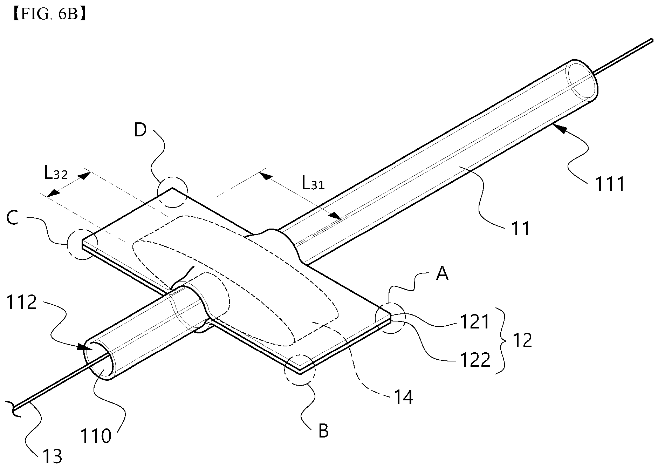

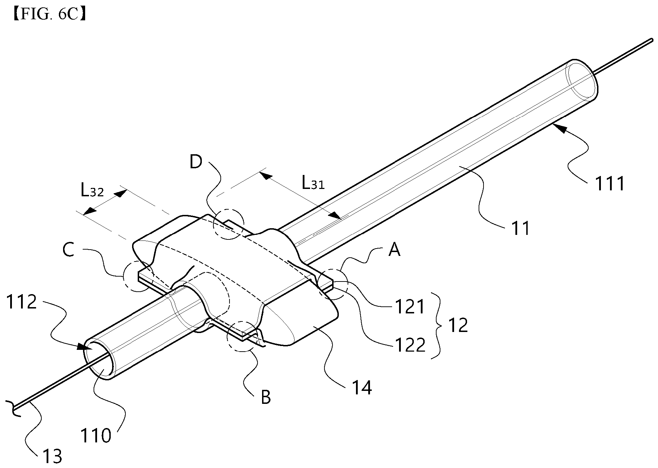

In the embodiment of FIG. 6A, the length L₃₁ of the wing 14 extending in the lateral direction of the tube 11 is almost the same as a length of the matrix 12. However, this is only an example, and according to embodiments, the length L₃₁ of the wing 14 may be determined so that the wing 14 protrudes more than the matrix 12 in the lateral direction of the tube 11 as shown in FIG. 6B, or the wing 14 protrudes less than the matrix 12 in the lateral direction of the tube 11 as shown in FIG. 6C. When the matrix 12 completely covers a side surface of the wing 14 as shown in FIG. 6B, the upper and lower sheet members 121, 122 may be adhered to each other not only at the vertices A, B, C, D but also at side edges, that is, the edges AB and CD, between the vertices, thereby promoting coupling stability.

Furthermore, although the length L₃₁ of the wing 14 extending in the lateral direction of the tube 11 changes in various ways in the embodiments of FIGS. 6A to 6C, in another embodiment, the wing 14 and the matrix 12 may be arranged so that the wing 14 protrudes more or less than the matrix 12 in the longitudinal direction of the tube 11 by changing the length L₃₂ of the wing 14 in the longitudinal direction of the tube 11.

Also, in FIGS. 6A to 6C, the matrix 12 coupled to the wing 14 is spaced apart from the proximal end 112 of the tube 11. However, this is only an example, and according to an embodiment, as a position of the wing 14 changes and/or a size of the matrix 12 changes, even when the wing 14 is present, an arrangement of the matrix12 with respect to the proximal end 12 of the tube 11 may change.

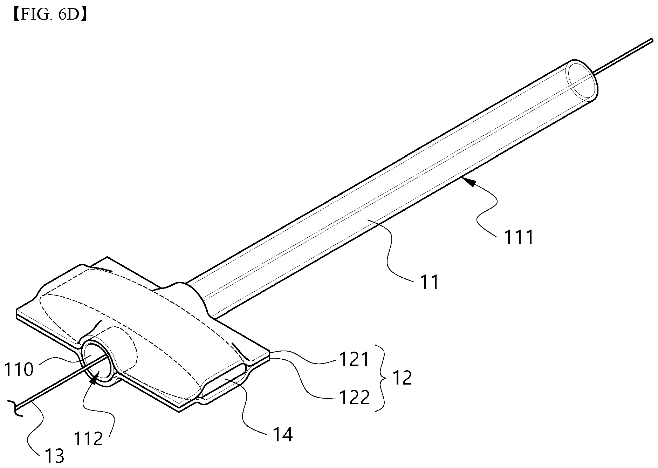

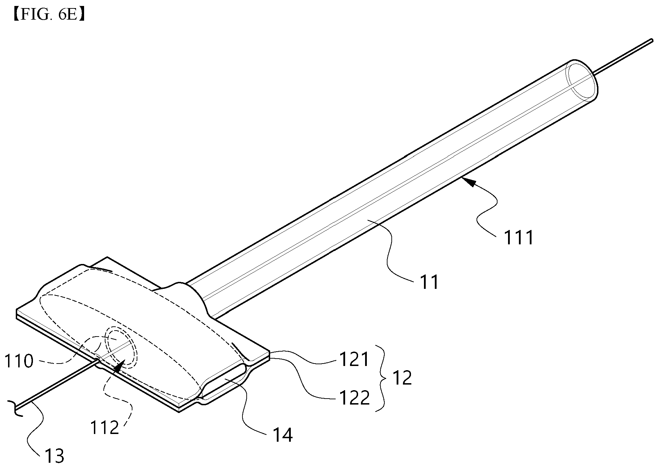

For example, as shown in FIG. 6D, the wing 14 may be disposed adjacent to the proximal end 112 compared to the embodiment of FIG. 6A and an end of the matrix 12 may contact the proximal end 112 of the tube 11, or as shown in FIG. 6E, the wing 14 may contact the proximal lend 112 of the tube 11 and an end of the matrix 12 may extend beyond the proximal end 112. Furthermore, the matrix 12 may be coupled to the wing 14 in an arbitrary arrangement not shown in the drawings of the specification.

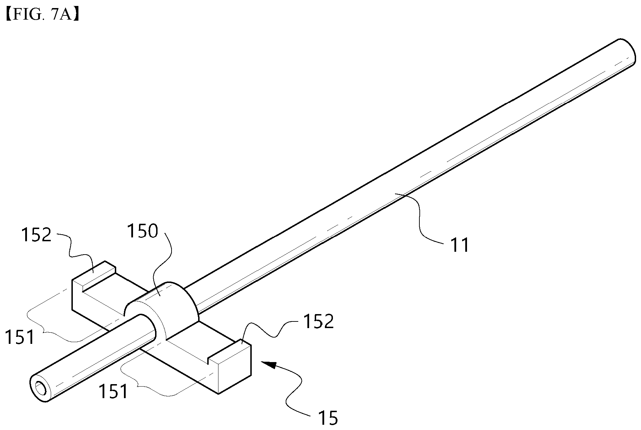

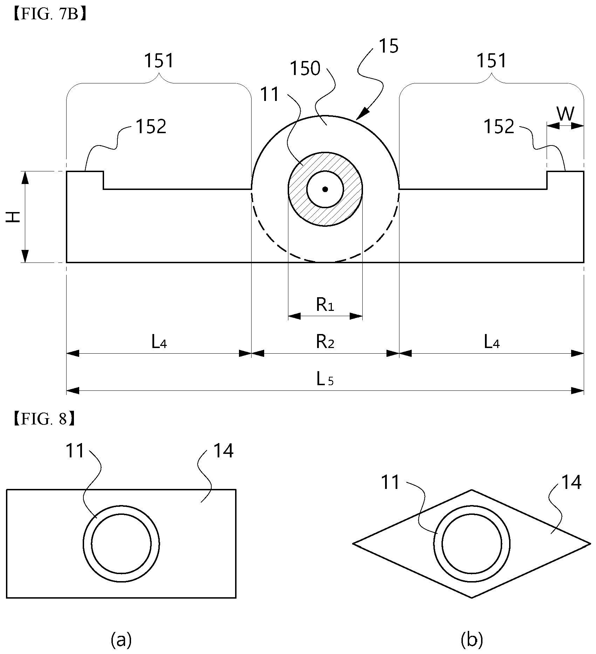

FIG. 7A is a perspective view illustrating a tube according to still another embodiment of the present disclosure. FIG. 7B is a cross-sectional view illustrating the tube of FIG. 7A. FIGS. 7A and 7B illustrate still another configuration of forming a wing protruding in a lateral direction of a tube in an implant device for an eye disease.

Referring to FIGS. 7A and 7B, in the present embodiment, the tube 11 is coupled to a support 15 extending in a direction different from a longitudinal direction of the tube 11. For example, the tube 11 may be coupled to the support 15 by passing through a fixing hole formed in a central portion 150 of the support 15 having a circular cross-section. The support 15 extends in a direction different from the longitudinal direction of the tube 11 to intersect the tube 11, and a part of the support 15 extending outward from an outer surface of the tube 11 corresponds to a wing 151.

Like the wing 14 described with reference to FIGS. 6A to 6E, the wing 151 according to the present embodiment prevents the tube 11 from being completely inserted into an anterior chamber of an eyeball and provides coupling stability with a matrix (not shown). That is, although not shown in FIGS. 7A and 7B, the matrix for impregnating and releasing a drug may also be coupled to the tube 11 of the implant device for an eye disease according to the present embodiment. Also, the matrix may be coupled to the tube 11 by being coupled to the wing 151 of the implant device according to the present embodiment. This may be easily understood from the embodiments described with reference to FIGS. 6A to 6E, and thus, a detailed description thereof will be omitted.

In an embodiment, a protrusion 152 is formed on a portion of the wing 151. The protrusion 152 is a protruding portion compared to other portions of the wing 151, that is, a portion of the support 15 having a larger width than other portions. For example, the protrusion 152 may be formed at an end of each of the wings 151 at both ends of the tube 11. When the wing 151 is additionally fixed with a thread according to an embodiment if necessary, the protrusion functions as an uneven portion for preventing the fixing thread from slipping.

In an embodiment, an outer diameter R₁ of the tube 11 (i.e., a diameter of the fixing hole of the support 15) is about 0.2 mm, and an outer diameter R₂ of the central portion 150 of the support 15 in which the fixing hole is formed is about 0.4 mm. Also, in an embodiment, a length L₅ of the support 15 is about 1.4 mm, a height H of the support 15 including a height of the protrusion 150 is about 0.25 mm, and a width W of the protrusion 150 is about 0.1 mm. A length L₄ of each of the wings 151 formed at both ends of the central portion 150 of the support 15 is 0.5 mm.

However, these numerical values are merely examples, and dimensions of portions of the tube 11 and the support 15 may vary according to embodiments and are not limited to the numerical values described in the specification.

According to the present embodiment, the wing 151 may be easily formed by fixing the tube 11 to the support 15 intersecting the tube 11, without having to attach or fix a member for forming a wing to the outer surface of the tube 11. The tube 11 on which the wing 151 is formed may be disposed so that an operator exfoliates conjunctival tissue or Tenon's tissue of the eyeball and then inserts it into the anterior chamber of the eyeball through exfoliated conjunctival tissue or Tenon's tissue.

Also, the tube 11 according to the present embodiment may be inserted into the eyeball through an injector as described with reference to FIG. 5, and in this case, the support 15 may be formed of a flexible material that may be inserted into a needle 21 of the injector 20 by being deformed when pressure is applied. For example, the support 15 may be pressed or rolled into close contact with the tube 11 and may be pushed into the needle 21. When the tube 11 is injected into the eyeball by a clinician's manipulation of the injector 20, the tube 11 and the support 15 may exit the injector through a front end of the needle 21, and the support 15 may be unfolded to function as the wing 151.

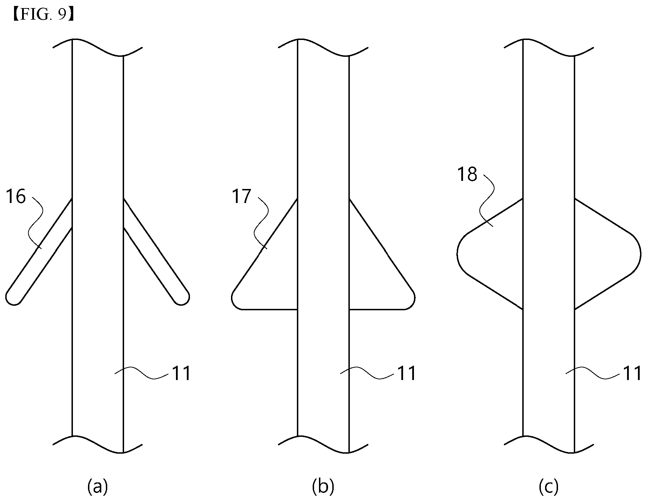

Furthermore, a shape of the wing described with reference to FIGS. 6A to 7B is only an example, and the wing of the tube in the implement device for an eye disease according to embodiments may have a cross-sectional shape or a planar shape different from that described above. FIGS. 8 and 9 illustrate some exemplary and non-limiting embodiments of a wing applicable to an implant device for an eye disease.

FIG. 8 is a cross-sectional view illustrating a shape of a wing in an implant device for an eye disease according to embodiments. In the embodiment described with reference to FIGS. 6A to 6E, the wing 14 is formed by using another tube-shape member surrounding the tube 11 and has a substantially elliptical cross-section. However, this is only an example, and the wing 14 may have a quadrangular cross-section as shown in (a) of FIG. 8, may have a rhombus cross-section as shown in (b) of FIG. 8, or may have any of other cross-sections not shown in the drawings of the specification.

Also, FIG. 9 is a plan view illustrating a shape of a wing in an implant device for an eye disease according to embodiments. In the implant device for an eye disease according to embodiments, the wing may be coupled to a tube in various ways. For example, the wing may be integrally manufactured with the tube, or may be manufactured separately from the tube and then may be attached to the tube.

Referring to (a) of FIG. 9, a wing 16 may be coupled to a side surface of the tube 11 in a direction orthogonal or inclined to a longitudinal direction of the tube 11. In this case, an inclination angle between the side surface of the tube 11 and the wing 16 may increase from a distal end of the tube 11 toward a proximal end of the tube 11, or conversely may increase from the proximal end of the tube 11 toward the distal end of the tube 11. When the angle of the wing 16 increases toward the distal end of the tube 11, because the wing 16 is inclined in a direction opposite to a direction in which the tube 11 is inserted into a sclera, even when the tube 11 moves toward an eyeball after the implant device is inserted, the wing 16 may be easily caught on the sclera to prevent the tube 11 from being completely inserted into the sclera.

Also, referring to (b) of FIG. 9, a wing 17 may have a tapered shape whose protruding length from a side surface of the tube 11 changes according to a position. For example, the wing 17 may have a tapered shape whose length increases from the proximal end toward the distal end of the tube 11, or may have a tapered shape whose length increases from the distal end toward the proximal end of the tube 11. When a length of the wing 17 increases toward the distal end of the tube 11, because the wing 17 has a maximum length at an end of an eyeball direction, even when the tube 11 moves toward the eyeball after the implant device is inserted, the wing 17 may be easily caught on the sclera to prevent the tube 11 from being completely inserted into the sclera.

Furthermore, referring to (c) of FIG. 9, a wing 18 may have a tapered shape whose length is maximized at a middle portion rather than at both ends of the wing 18 and whose length decreases toward both ends of the wing 18.

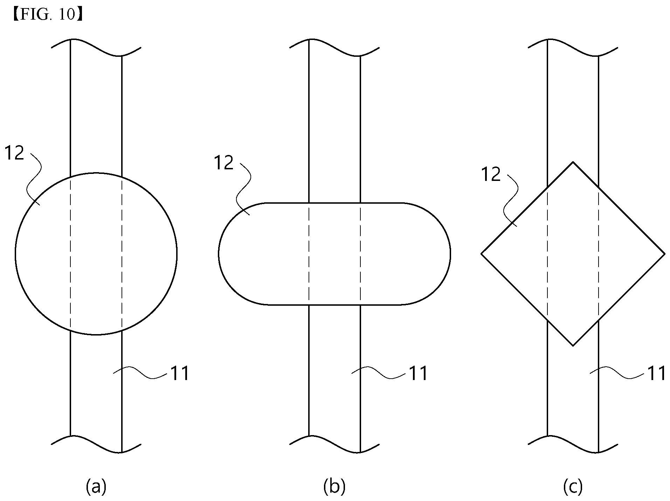

FIG. 10 is a plan view illustrating a shape of a matrix in an implant device for an eye disease according to embodiments.

In the embodiments described with reference to FIGS. 3A to 9, the matrix 12 is a member having a quadrangular sheet shape. However, this is only an example, and the matrix 12 according to embodiments may have any of various shapes. For example, the matrix may have a circular shape as shown in (a) of FIG. 10, an elliptical shape as shown in (b) of FIG. 10, or a rhombus shape as shown in (c) of FIG. 10. For example, in another embodiment, the matrix 12 may have a shape that is inclined with respect to the tube 11 or a tapered shape, like the wings 16 to 18 of (a) to (c) of FIG. 9.

Although a shape of the matrix 12 coupled to the tube 11 including no wing is illustrated in FIG. 10, it will be understood by one of ordinary skill in the art that the matrix 12 having any of various shapes may be applied to the tube 11 including a wing.

The above description of the present disclosure is provided for illustration, and it will be understood by one of ordinary skill in the art that various changes in form and details may be readily made therein without departing from essential features and the scope of the present disclosure. Accordingly, the above embodiments are examples only in all aspects and are not limited. For example, each component described as a single type may be executed in a distributed manner, and components described as a distributed type may be executed in a combined manner.

The scope of the present disclosure is indicated by the claims rather than by the detailed description of the present disclosure, and it should be understood that the claims and all modifications or modified forms drawn from the concept and scope of the claims and equivalents are included in the scope of the present disclosure.

### [Industrial Applicability]

The present disclosure relates to an implant device for an eye disease, and more particularly, to an implant device for an eye disease in which a matrix configured to temporarily absorb a drug is coupled to a tube of the implant device, so that in a state where the implant device is inserted into an eyeball, the drug such as an antifibrotic agent is released from the matrix to surrounding tissue.

## Claims

1. An implant device for an eye disease to be inserted into an eyeball, the implant device for an eye disease comprising:
a tube having one end to be inserted into an anterior chamber of the eyeball, the tube comprising a hollow portion through which aqueous humor is drained; and
a matrix coupled to an outer surface of the tube and formed of a material capable of impregnating and releasing a drug,
wherein at least a part of the matrix has a cross-section larger than a diameter of the tube.

2. The implant device for an eye disease according to claim 1, wherein the tube comprises:
a body extending in one direction; and
a wing integrally formed with the body or detachably coupled to the body and extending in a lateral direction of the body,
wherein the matrix is at least partially coupled to the wing.

3. The implant device for an eye disease according to claim 1, wherein the matrix comprises a pair of sheet members,
wherein the tube is disposed between the pair of sheet members.

4. The implant device for an eye disease according to claim 3, wherein edges or vertices of the pair of sheet members are at least partially adhered to each other.

5. The implant device for an eye disease according to claim 1, wherein the matrix extends in a direction different from a longitudinal direction of the tube to prevent the tube from being completely inserted into a sclera of the eyeball.

6. The implant device for an eye disease according to claim 5, wherein the matrix is coupled to the tube to protrude in a lateral direction of the tube at a position spaced apart from a distal end of the tube by a preset distance.

7. The implant device for an eye disease according to claim 1, wherein the matrix comprises any one material selected from the group consisting of poly(acrylic acid), polyacrylamide, poly(sulfopropyl acrylate), poly(2-hydroxyethyl methacrylate), poly(vinyl alcohol), silicone, polyurethane, collagen, gelatin, hyaluronic acid, poly(aspartic acid), alginate, hydroxypropyl cellulose, hydroxypropyl methylcellulose acetate succinate, and chitosan, or a combination thereof.

8. The implant device for an eye disease according to claim 1, wherein the matrix comprises a first material that is not biodegradable in the eyeball and a second material capable of temporarily absorbing a drug.

9. The implant device for an eye disease according to claim 8, wherein the first material comprises any one material selected from the group consisting of silicone, polyethylene vinyl acetate, polyvinyl acetate, polycarbonate, polyvinyl chloride, polyurethane, poly(methyl methacrylate), poly(butyl methacrylate), polyethylene, polypropylene, polyethylene terephthalate, glycol-modified polyethylene terephthalate, polytetrafluoroethylene, polyhydroxyalkanoates, parylene, polyether ether ketone, polyimide, epoxy-based resin, poly(vinylidene fluoride), polyether block amides, 3-[tris(trimethylsiloxy)silyl]propyl methacrylate, poly(styrene-b-isobutylene-b-styrene) copolymer, cellulose acetate, poly((N-vinylpyrrolidone)-block-poly(vinyl acetate)), and ethyl cellulose, or a combination thereof.

10. The implant device for an eye disease according to claim 8, wherein the second material comprises any one material selected from the group consisting of poly(ethylene glycol), poly(ethylene glycol) derivatives, poly(ethylene oxide), poly(propylene oxide), polyvinyl alcohol, polyvinyl pyrrolidone, polyethersulfone, polyamide, polyacrylamide, polyglycolic acid, polyacrylic acid, glucuronic acid, hexuronic acid, hyaluronic acid, polyaspartic acid, alginate, polyorthoester, 2-hydroxyethyl methacrylate, polycaprolactone, polylactic acid, poly(lactide-co-glycolide), polydioxanone, polyhydroxybutyrate, polyhydroxyalkanoates, collagen, gelatin, hydroxypropyl cellulose, and chitosan, or a combination thereof.

11. The implant device for an eye disease according to claim 1, wherein the matrix is configured to impregnate a drug comprising at least one material selected from the group consisting of dexamethasone, mitomycin-C, 5-fluorouracil, triamcinolone, an anti-vascular endothelial growth factor agent, and a transforming growth factor-beta inhibitor, or a combination thereof.
